(19) Europäisches Patentamt
European Patent Office
Office européen des brevets

(11) Publication number: **0 167 202 B1**

(12) **EUROPEAN PATENT SPECIFICATION**

(45) Date of publication of patent specification: **31.01.90**

(51) Int. Cl.$^5$: **C 07 C 53/50,** C 07 C 51/62

(21) Application number: **85200976.0**

(22) Date of filing: **19.06.85**

(54) **Alpha chlorination process employing food grade antioxidants, oxygen or cyanoquinones**

(30) Priority: **29.06.84 US 625938**
**29.06.84 US 625922**
**29.06.84 US 625921**

(43) Date of publication of application:
**08.01.86 Bulletin 86/02**

(45) Publication of the grant of the patent:
**31.01.90 Bulletin 90/05**

(84) Designated Contracting States:
**BE DE FR GB IT NL**

(56) References cited:
**CH-A- 601 164**
**US-A-3 751 461**
**US-A-4 148 811**
**US-A-4 368 140**

(73) Proprietor: **THE PROCTER & GAMBLE COMPANY**
**One Procter & Gamble Plaza**
**Cincinnati Ohio 45202 (US)**

(72) Inventor: **Thompson, James Edwin**
**9897 Lorelei Drive**
**Cincinnati Ohio 45231 (US)**

(74) Representative: **Brooks, Maxim Courtney et al**
**Procter & Gamble European Technical Center**
**Temselaan 100**
**B-1820 Strombeek-Bever (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 167 202 B1

Courier Press, Leamington Spa, England.

## Description

The present invention relates to improved processes for selectively chlorinating acyl compounds at the alpha or 2-position.

### BACKGROUND OF THE INVENTION

The halogenation of organic compounds at a specific site or position (regiospecific halogenation) is a very difficult task. In general, hydrocarbon compounds tend to undergo halogenation to some degree at virtually every available carbon-hydrogen linkage. The random nature of this substitution generally leads to an undesirable mixture of isomers and homologs. Further, when additional reactants are employed to make the reaction more selective or specific, additional competing reactions are also likely to be encountered; when more reactants are employed there is a greater likelihood of side or competing reaction simply because there are more components in the system.

The present invention makes use of the discovery that the most efficient way to prepare alpha-substituted acyl compounds, particularly alpha-chloro acyl chlorides and acyl esters, is to begin with the corresponding carboxylic acid chloride.

The Hell-Volhard-Zelinksy (HVZ) alpha-bromination of carboxylic acids was discovered a century ago. This was based upon the observation that in the presence of a small amount of phosphorus, aliphatic carboxylic acids react smoothly with bromine to yield a compound in which the alpha-hydrogen has been replaced by a bromine substituent. Only recently has work related to regiospecific halogenation of acyl compounds focused upon the alpha-substitution of halogens other than bromine, and in particular to extending the HVZ reaction to alpha-chloro substitution. It was then quickly appreciated that the propensity of chlorine to undergo competing free radical reactions under HVZ conditions required the development of specialized procedure that would favor the ionic alpha-substitution process. See Little, Saxton, et. al., *Journal of the American Chemical Society 91* 7098 (1969); US—A—3,584,036 to Saxton, et. al., both of which relate to the solution to this problem.

Ogata, et. al., have developed a procedure which employs the addition of gaseous chlorine to a neat aliphatic acid at 140°C in the presence of a strong acid catalyst and oxygen. Ogata suggests that the preferred catalyst is chlorosulfonic acid (10 mol % of the carboxylic acid) in combination with the oxygen gas. See Ogata, et. al., *J. Org. Chem., 40* 2960 (1975); Ogata, et. al., *Tetrahedron 26* 5929 (1970); Ogata, et. al., *Nippon Kagaku Kaishi* 1517 (1975) [Chem. Abstr. 83 178239 (1975)]; Ogata, et. al Japanese Patent 75—135024; Ogata et. al., Japanese Patent 74—24913. This method works particularly well for short chain ($C_2$—$C_6$) carboxylic acids. However, for chain lengths greater than $C_6$, the reaction is adequate, but sub-optimal. There is no suggestion here of halogenating acyl halide starting materials.

Crawford, US—A—4,148,811, issued April 10, 1979; US—A—4,368,149, issued January 11, 1983; *J. Org. Chem.* 48 1364 (1983); describes the regiospecific halogenation of long chain, as well as short chain, carboxylic acids, carboxylic acid halides, and acid anhydrides, employing a modification of Ogata's reaction. The major change is the use of a free radical inhibitor, such as 7,7,8,8-tetracyanoquinodimethane (TCNQ), in place of the oxygen gas. This change (TCNQ at 0.5 mol % in place of the oxygen) resulted in an improved reaction that was unaffected by chain length. (As stated above the use of oxygen and chloranil as taught by Ogata does not perform well on chain length greater than about $C_6$.) While Crawford states that this system can be used with the carboxylic acid, acid halide and acid anhydride, he states that temperatures above 130°C must be employed if elemental chlorine is used as the halogen source. He also describes the many quinone or "quinone-like" structures which can be used as free radical inhibitors.

US—A—3,880,923, Scheider, et al., issued April 29, 1975, describes an improved process for the production of alpha-chloro-carboxylic acid chlorides by reacting a carboxylic acid chloride with chlorine at an elevated temperature in the presence of sulfuric acid.

US—A—4,169,847, Degischer, issued October 2, 1979, describes an improved process for the production of alpha-chloro-alkanoyl chlorides by reacting alkanoyl chlorides with chlorine at an elevated temperature in the presence of chlorosulphonic acid as a catalyst.

US—A—3,152,055, Katzschmann, issued October 6, 1964, describes production of aromatic chloro-carboxylic acid chlorides by using an ester as a starting material.

The methods of the present invention are simpler and more efficient, and possesses the following advantages versus those described in the art-disclosed processes: The reaction takes place under less rigorous conditions particularly when using elemental chlorine, goes more quickly, yields less alpha, alpha-dichloro material (and other products of competing reactions), and works efficiently regardless of the chain length of the starting material.

In particular, the present invention provides a marked improvement over the processes disclosed in US—A—3,584,036; US—A—1,148,811; and US—A—4,368,140; discussed above.

With respect to the processes described by Crawford, the present method allows the use of elemental chlorine at temperatures significantly below 130°C. This is accomplished by employing the carboxylic acid halide, particularly the carboxylic acid chloride, as the starting material. Certain of the present methods allow excellent results to be obtained by employing commercial antioxidants in place of the more expensive free radical inhibitors of Crawford if the carboxylic acid chloride is used as a starting material;

elemental chlorine and lower temperatures may also be employed.

With respect to the processes described by Ogata, the present method is made more efficient by beginning with the carboxylic acid halide, particularly the carboxylic acid chloride. This means that significantly less rigorous conditions may be employed. For example, if chloranil is employed as a free radical inhibitor in a process which begins with the free acid, and in a process which begin with the acid chloride, a higher yield can be obtained with a lower reaction temperature by employing the acid chloride (i.e., by following the teachings of the present invention).

The same is true if air or elemental oxygen are employed. If the acid chloride is used as the starting material and air or elemental oxygen are employed alone (without the free radical inhibitors employed by Ogata and Crawford, chloranil or TCNQ), the methods above can be run with equivalent results.

## SUMMARY OF INVENTION

The process of the present invention comprises a method of producing alpha-chloro acyl chlorides comprising contacting an acyl chloride with a chlorine source at a temperature of about 75°C to about 125°C in the presence of (i) an effective amount of an auxiliary acidic agent and (ii) an effective amount of a free radical inhibitor selected from the group consisting of food grade antioxidants, oxygen, a cyanoquinone material and mixtures thereof. The acyl chlorides, food grade antioxidant and cyanoquinone are further defined hereinbelow.

## DETAILED DESCRIPTION OF THE INVENTION

The present invention encompasses an improved process for chlorinating acyl halides. The acyl chlorides used herein are characterized in that they contain at least one reactive hydrogen substituent at the alpha or 2-position. It is this hydrogen substituent which is displaced during the process of the present invention.

In the practice of this invention employing cyanoquinones, the acyl chloride is contacted with a chlorine source in the presence of an effective amount of an acidic auxiliary agent and an effective amount of a cyanoquinone material. This process is carried out at a temperature of 75°C to 125°C.

In the practice of this invention employing antioxidants, the acyl chloride is contacted with a chlorine source in the presence of an effective amount of an acidic auxiliary agent and an effective amount of a food grade antioxidant material. This process is carried out at a temperature of 75°C to 125°C.

In the practice of this invention employing air or oxygen alone, the acyl chloride is contacted with a chlorine source in the presence of an effective amount of an acidic auxiliary agent and air or elemental oxygen. The process is carried out at a temperature of 75°C to 125°C.

The methods of the present invention may be employed to replace the alpha or 2-hydrogen substituent of any acyl halide with any halogen. However, as is well known, bromination, iodination, and particularly fluorination, reactions must be carried out under special conditions. Such reactions are therefore not contemplated in the practice of the present invention. Thus, while the present invention is particularly useful for both chlorinating and brominating acyl halides, on an industrial scale the processes of the present invention are particularly useful for chlorination reactions.

Thus, by the term "chlorination" or "chlorinating" herein is meant replacing the alpha or 2-hydrogen substituent of any acyl chloride with a chlorine; however, the processes herein are useful for conducting bromination reactions.

By the term "acyl chloride" herein is meant compounds of the formula

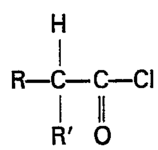

wherein R and R′ are H or the same or different hydrocarbyl substituents having from 1 to 28 carbon atoms.

The processes of the present invention are particularly useful in chlorinating the acid (acyl) chlorides of octanoic, nonanoic, decanoic (capric), dodecanoic (lauric), tetradecanoic (myristic), hexadecanoic (palmitic), and octadecanoic (stearic) acids.

By the term "effective amount" of some material, component or agent, as that term is used herein, is meant a sufficient amount of that material, component or agent is present to direct the chlorination reaction regiospecifically such that it occurs almost exclusively at the alpha or 2-position of the acyl chloride.

By "comprising" herein is meant that various other compatible materials may be present in the reaction mixtures during the chlorination process. These materials and their levels may be selected such that they do not adversely affect regiospecificity of the chlorination reaction. For example, various solvents or solvent mixtures may be optionally employed.

All percentages used herein are on a mol basis, unless otherwise specified. Mixture of any component may be used.

## ACIDIC AUXILIARY AGENTS

The acidic auxiliary agents used in the practice of the present invention include many of the common acidic materials known in the art for use in chlorination reactions. Such materials include both Lewis acids and inorganic protic acids which are stronger acids than hydrocarbyl carboxylic acids. Such acidic auxiliary agents include, without limitation, $PBr_3$, $PCl_3$, thionyl chloride, sulfuryl chloride, oxalyl chloride, sulfonyl chloride, $PCl_5$, phosgene, fluorosulfonic acid, chlorosulfonic acid, and trifluoromethanesulfonic acid. Chlorosulfonic acid is preferred.

It will be appreciated that the acyl halides themselves are not operative in the practice of this invention as acidic auxiliary agents inasmuch as it would then require the separation of the alpha-chlorinated acidic auxiliary agent from the material undergoing the desired alpha-halogenation.

## CYANOQUINONES

The cyanoquinone materials used herein are selected from cyanoquinones, hexacyanobutadiene, and mixtures thereof and are all members of a well-known class of compounds. They have been associated with the synthesis of alpha-halogenated fatty acids, and have also been the subject of intensive study as electrically-conductive organic solids. Reviews of the preparation and properties of these materials and related materials can be found in the articles by Wheland, et. al., *J. Amer. Chem. Soc. 98* 3916 (1976); and Wheland et. al., *J. Org. Chem. 40* 3101 (1975).

The use of these materials as free radical inhibitors is described in detail in US—A—4,148,811, issued April 10, 1979; US—A—4,368,149, issued January 11, 1983; J. Org. Chem. 48 1364 (1983).

A particularly useful synthesis is described by Crawford in "A Practical Synthesis of 7,7,8,8-Tetracyanoquinodimethane", *J. Org. Chem. 48,* 1366 (1983).

In general, the cyanoquinones useful herein are characterized by the moiety

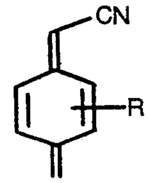

where R can be H or one or more substituent groups, e.g., halogen, alkyl, alkoxy, thioalkyl, CN, etc., as described by Wheland, et. al., above.

The preferred cyanoquinones are the tetracyanoquinodimethane ("TCNQ") compounds of the formula

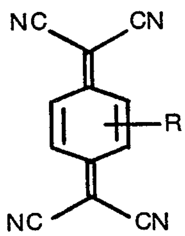

where R is as above.

Highly preferred cyanoquinone materials useful in the processes of the present invention include 7,7,8,8-tetracyanoquinodimethane ("TCNQ"), hexacyanobutadiene ("HCBC") and tetracyanonaphthoquinodimethane ("TNAP"). These can be depicted as follows:

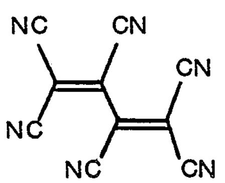

HCBD

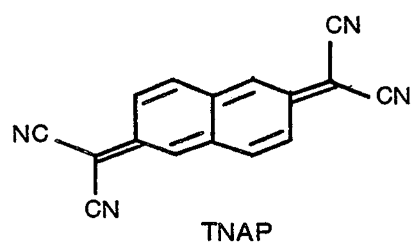

TNAP

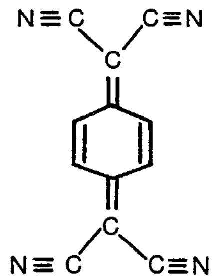

TCNQ

Tetracyanoquinodimethane ("TCNQ"), hexacyanobutadiene ("HCBC") and tetracyanonaphthoquinodimethane ("TNAP") can all be prepared according to conventional, art-disclosed techniques, including those discussed by Wheland, et al., as described above.

It should be noted that HCBD is not, in a technical sense, a formal quinone structure. However, its extended conjugated system of electrons gives it "quinone-like" characteristics. It has therefore come to be considered a cyanoquinone in the art; it is so considered within the present invention.

The most preferred catalyst system herein comprises a mixture of TCNQ and chlorosulfonic acid. Various ratios of these materials can be employed, but a ca. 1:5—1:50 mole ratio of TCNQ:$ClSO_3H$ is convenient.

The most preferred chlorination reagent herein comprises gaseous or liquid elemental chlorine and the aforesaid mixture of TCNQ and chlorosulfonic acid. Of course, the chlorine in this reagent is replenished as it is exhausted during the reaction. Replenishment of the chlorine is most conveniently carried out by bubbling gaseous chlorine into the reaction mixture.

In a highly preferred embodiment of the present invention, the above catalyst system is used in combination with air or elemental oxygen. Air or elemental oxygen is bubbled thorugh the reaction mixture preferably at a rate which achieves saturation in the reaction mixture.

The chlorination reaction of this invention is carried out by contacting the acyl chloride compound with the chlorine or chlorine source in the presence of a cyanoquinone material and acidic auxiliary agent at a temperature of 75°C to 125°C.

In particular, chlorination reactions using elemental chlorine as the chlorine are carried out at temperatures of 75°C to 125°C, preferably at temperatures with the range from 100°C to 110°C. Bromination and iodination reactions are carried out under similar temperature conditions.

The process herein can be carried out in the presence or absence of inert solvents. Preferably, the reaction is carried out without the use of solvents, as this is both convenient and economical on a commercial scale. Indeed, the use of solvents can lead to undesired side-reactions involving chlorination of many of the common hydrocabron solvents. Under the reaction temperatures specified hereinabove, the acyl chloride compounds are liquids and are quite convenient to use in that state without additional solvents.

Typical use concentrations of the cyanoquinones relative to the acyl chloride compounds are 0.01—10.0 mole percent, preferably 0.05—5.0 mole percent, most preferably 0.05—0.5 mole percent.

Typical use concentrations of the acidic auxiliary agents relative to the acyl chloride compounds are 0.1—10 mole percent, preferably 1.0—5 mole percent.

## ANTIOXIDANTS

It has also been discovered that certain antioxidants can be employed in the reaction of the present invention. Since many antioxidants work by inhibiting or interrupting the propagation step in the oxidation process, it was postulated (and confirmed) that these same agents would work in the same fashion, i.e., as free radical inhibitors, in the processes of the present invention.

Food antioxidants for use herein are selected from butylated hyroxyanisole (BHA), butylated hydroxytoluene (BHT), t-butyl hydroquinone (TBHQ), propyl gallate (PG), and 6-ethoxy-1,2-dihydro-2,2,4-trimethylquinoline, and mixtures thereof.

Particularly preferred antioxidants include butylated hydroxyanisole (BHA), butylated hydroxytoluene (BHT), and propyl gallate (PG). Mixtures of all the above may also be employed.

The preferred catalyst systems herein comprise mixtures of TBHQ, BHA, or BHT, and chlorosulfonic acid. Various ratios of these materials can be employed, but a 1:5—1:50 mole ratio of antioxidant:$ClSO_3H$ is convenient.

The most preferred chlorination reagent herein comprises gaseous or liquified elemental chlorine and the aforesaid mixtures of antioxidant and chlorosulfonic acid. Of course, the chlorine in this reagent is replenished as it is exhausted during the reaction. Replenishment of the chlorine is most conveniently carried out by bubbling gaseous chlorine into the reaction mixture.

The chlorination reaction of this invention is carried out by contacting the acyl chloride compound with the chlorine or chlorine source in the presence of material and acidic auxiliary agent at a temperature of 75°C to 125°C.

In a highly preferred embodiment of the present invention, the above catalyst system is used in

combination with air or elemental oxygen. Air or elemental oxygen is bubbled through the reaction mixture preferably at a rate which achieves saturation in the reaction mixture.

In particular, chlorination reactions using elemental chlorine as the chlorine are carried out at temperatures of 75°C to 125°C, and preferably at temperatures with the range from 100°C to 110°C. Bromination and iodination reactions are carried out under similar temperature conditions.

The process herein can be carried out in the presence or absence of inert solvents. Preferably, the reaction is carried out without the use of solvents, and this is both convenient and economical on a commercial scale. Indeed, the use of solvents can lead to undesirable side-reactions involving chlorination of many of the common hydrocarbon solvents. Under the reaction temperatures specified hereinabove, the acyl chloride compounds are liquids and are quite convenient to use in that state without additional solvents.

Typical use concentrations of the antioxidants relative to the acyl chloride compounds are 0.1—10.0 mole percent, preferably 0.1—5.0 mole percent, most preferably 0.5—0.5 mole percent.

Typical use concentrations of the acidic auxiliary agents relative to the acyl chloride compounds are 0.1—10 mole percent, preferably 1.0—2.5 mole percent.

The following examples illustrate the practice of the present invention but are not intended to be in any way limiting thereof.

AIR/OXYGEN ALONE

As has been described above in detail, it has been discovered that many known alpha chlorination reactions can be improved (better yields under less rigorous conditions) by using acyl chloride starting materials. It has also been discovered that the increased efficiency which results from the methods employing acyl chloride starting materials allows this reaction to be conducted just as efficiently as known methods (Ogata, Crawford, discussed above) without the use of antioxidants or cyanoquinones.

Accordingly, the process of the present invention comprises a method of producing alpha-chloro acyl chlorides in the absence (less than 0.005 mol%) of antioxidants or cyanoquinones comprising contacting an acyl chloride with a chlorine source at a temperature of 75°C to 125°C in the presence of (i) an effective amount of an auxiliary acidic agent and (ii) an effective amount of elemental oxygen or air. (See Trial 14 of Example IV.)

The preferred catalyst systems herein comprises a mixture of air and chlorosulfonic acid. Various ratios of these materials can be employed, but a system which passes air through the reaction mixture such that saturation is achieved is preferred and convenient.

The most preferred chlorination reagent herein comprises gaseous or liquid elemental chlorine and the aforesaid mixtures of air or oxygen and chlorosulfonic acid. Of course, the chlorine in this reagent is replenished as it is exhausted during the reaction. Replenishment of the chlorine is most conveniently carried out by bubbling gaseous chlorine into the reaction mixture.

The chlorination reaction of this invention is carried out by contacting the acyl chloride compound with the chlorine or chlorine source in the presence of material and acidic auxiliary agent at a temperature of 75°C to 125°C.

In particular, chlorination reactions using elemental chlorine as the chlorine are carried out at temperatures of 75°C to 125°C, and preferably at temperatures with the range from 100°C to 110°C. Bromination and iodination reactions are carried out under similar temperature conditions.

The process herein can be carried out in the presence or absence of inert solvents. Preferably, the reaction is carried out without the use of solvents, and this is both convenient and economical on a commercial scale. Indeed, the use of solvents can lead to undesirable side-reactions involving chlorination of many of the common hydrocarbon solvents. Under the reaction temperatures specified hereinabove, the acyl chloride compounds are liquids and are quite convenient to use in that state without additional solvents.

The following examples illustrate the practice of the present invention but are not intended to be in any way limiting thereof.

Example I

Alpha-Chlorination of Nonanoyl Chloride in the Presence of Tetracyanoquinodimethane (TCNQ)

A 1.0 liter five-necked round-bottomed flask was fitted with a mechanical stirrer, thermometer, dry ice condenser (with a gas exit tube) and two fritted glass dispersion tubes which extended to near the bottom of the flask. The two dispersion tubes were connected via PVC tubing to a Y connector and thence to a chlorine source. An in-line flow meter was employed.

The flask was charged with 487 g (2.75 moles) of nonanoyl chloride and was immersed in an oil bath maintained at 100°C. TCNQ (1.8 g, 8.8 millimoles, 0.32 mole %) and chlorosulfonic acid (9.6 g, 82.5 millimoles, 3 mole %) were added and the chlorine flow was started (time, t = 0), The flow rate was 2.5 liters/min (900 ml/min/mole).

A mild exotherm occurred which gradually raised the temperature to 120°C. it held there until near the end of the reaction. At t = 25 min, an additional 1.0 g (4.9 millimoles, 0.18 mole %) of TCNQ was added. At t = 40 min (T = 120°C), chlorine began to reflux more rapidly and the temperature began to fall. By t = 49 min, the temperature had dropped to 90°C and the chlorine flow was stopped. The oil bath was removed. Aliquots subsequently showed that the reaction was complete by t = 45 min.

The reaction mixture was degassed and the alpha-chloro acid chloride was distilled directly from the reaction flask. A main fraction, 494 g (85%), b.p. 69—70°C (0.5 mm) was obtained. This fraction was 99% pure by gas chromatography. Other, less pure distillation fractions contained an additional 5—6% of the desired product.

Substantially similar results are obtained when the above reaction is conducted replacing the TCNQ, in whole or in part with hexacyanobutadiene ("HCBC") and tetracyanonaphthoquinodimethane ("TNAP").

The process of Example I is carried out using the acid (acyl) chlorides of octanoic, decanoic (capric), dodecanoic (lauric), tetradecanoic (myristic), hexadecanoic (palmitic), and octadecanoic (stearic) acids. Substantially similar results (high yields) are obtained.

Substantially similar results are obtained when the chlorosulfonic acid is replaced, in whole or in part, with $PBr_3$, PCl, thionyl chloride, sulfuryl chloride, oxalyl chloride, sulfonyl chloride, $PCl_5$, phosgene, fluorosulfonic acid, and trifluoromethanesulfonic acid.

Example II

Alpha-Chlorination of Octanoyl Chloride in the Presence of Air and Tetracyanoquinodimethane (TCNQ)

A 100 ml four-necked round-bottomed flask equipment for magnetic stirring was fitted with a thermometer, dry ice condenser (with a gas exit tube) and two gas inlet tubes. The inlet tubes each had 3 mm diameter openings and extended to near the bottom of the reaction flask. One was connected via PVC tubing to a chlorine source containing an in-line flow meter. The other was similarly connected to a compressed air source.

The flask was charged with 52.2 g (320 millimoles) of octanoyl chloride and was immersed in an oil bath maintained at 100°C. When the acid chloride temperature reached 100°C, TCNQ (0.033 g, 0.16 millimoles, 0.05 mole %) and chlorosulfonic acid (1.2 g, 10 millimoles, 3.1 mole %) were added. Flows of air and chlorine, each at 400 ml/min (1250 ml/min/mole), were then begun (time t = 0).

The reaction temperature gradually rose to 116°C. At t = 25 min, chlorine reflux began to accelerate and the reaction was stopped at t = 30.5 min.

For convenient yield determination, the acid chloride was converted to methyl ester by the addition of 150 ml of methanol. This mixture was dissolved in 300 ml of methylene chloride and was washed successively with 100 ml portions of water (twice) saturated sodium bicarbonate solution (once) and water again (twice). The organic solution was dried overnight, filtered and distilled to give 57.0 g (93%) of distillate, b.p. 72° (0.3 mm). By gas chromatography, the product consisted of 97.7% monochloro- and 2.3% dichloro octanoate.

Substantially similar results are obtained when the TCNQ is replaced, in whole or in part, with hexacyanobutadiene ("HCBC") and tetracyanonaphthoquinodimethane ("TNAP").

The process of Example II is carried out using the acid (acyl) chlorides of nonanoic, decanoic (capric), dodecanoic (lauric), tetradecanoic (myristic), hexadecanoic (palmitic), and octadecanoic (stearic) acids. Substantially similar results (high yields) are obtained.

Substantially similar results are obtained when the chlorosulfonic acid is replaced, in whole or in part, with $PBr_3$, $PCl_3$, thionyl chloride, sulfuryl chloride, oxalyl chloride, sulfonyl chloride, $PCl_5$, phosgene, fluorosulfonic acid, and trifluoromethanesulfonic acid.

Example III

Alpha-Chlorination of Nonanoyl Chloride in the Presence of t-Butylhydroquinone (TBHQ) and Air

A 1.0 liter five-necked round-bottomed flask was fitted with a mechanical stirrer, thermometer, dry ice condenser (with a gas exit tube) and two fritted glass dispersion tubes which extended to near the bottom of the flask. One of the tubes was connected via PVC tubing to a chlorine source containing an in-line flow meter. The other was similarly connected to a compressed air source.

The flask was charged with 487 g (2.75 moles) of nonanoyl chloride and was immersed in an oil bath maintained at 100°C. When the temeprature of the acid chloride reached 100°C, TBHQ (1.3 g, 7.8 millimoles, 0.28 mole %) and chlorosulfonic acid (9.6 g, 82.5 millimoles, 3 mole %) were added. Flows of chlorine and air, each at 350 ml/min, were then begun (t = o).

A mild exotherm occurred which gradually raised the temperature to 124°C. It held there until near the end of the reaction. At t = 25 min, an additional 1.0 g (6.0 millimoles, 0.22 mole %) of TBNQ was added. At t = 9 min (T = 117°C), chlorine began to reflux more rapidly and the temperature began to fall. Gas flow was stopped and the oil bath was removed. An aliquot showed that the reaction mixture consisted of 92% alphachlorononanoyl chloride.

The reaction mixture was degassed and the alpha-chloro acid chloride was distilled directly from the reaction flask. A main fraction, 492 g (85%), b.p. 69—70°C (0.5 mm) was obtained. This fraction was 93% pure by gas chromatography. An additional 7—8% of the desired product was present in subsequent, less pure fractions.

Substantially similar results are obtained when the TBHQ is replaced, in whole or in part, with butylated hydroxyanisole (BHA), butylated hydroxytoluene (BHT), t-butyl hydroquinone (TBHQ), propyl gallate (PG), and 6-ethoxy-1,2-dihydro-2,2,4-trimethylquinoline.

The process of Example II is carried out using the acid (acyl) chlorides of octanoic, decanoic (capric),

dodecanoic (lauric), tetradecanoic (myristic), hexadecanoic (palmitic), and octadecanoic (stearic) acids. Substantially similar results (high yields) are obtained.

Substantially similar results are obtained when the chlorosulfonic acid is replaced, in whole or in part, with $PBr_3$, $PCl_3$, thionyl chloride, sulfuryl chloride, oxalyl chloride, oxalyl chloride, sulfonyl chloride, $PCl_5$, phosgene, fluorosulfonic acid, and trifluoromethanesulfonic acid.

In the following Example, the following abbreviations are employed. For reactants (starting materials), "NC" represents nonanoyl chloride; "OC" represents octanoyl chloride; "SC" represents stearoyl chloride; and "NA" represents nonanoic acid "CSA" is used for the auxiliary acidic agent chlorosulfonic acid.

Time is time in minutes. Products: Products A represents percent of alpha-chloro product; B represents percent of starting material (reactant) remaining; C represents the percent of alpha, alpha-dicloro material produced; D represents all other materials, including free radical product. A single asterisk (*) represents a flow rate for that gas of 625 ml/min/mol; (**) represents a rate of 1250 ml/min/mol; (***) represents a rate of 156 ml/min/mol. In the case of trials 2, 3, 4 and 17, additional amounts of TCNQ are added after the indicated times (minutes) have elapsed.

EXAMPLE IV

Chlorination of Acid Chlorides

| Trial | Reactant | $Cl_2$ | T°C | CSA | System | Time | Products A | B | C | D |
|---|---|---|---|---|---|---|---|---|---|---|
| 1 | NC | 625 | 150 | 3.1 | 0.5 TCNO | 40′ | 94 | 0 | 3.8 | 1.1 |
| 2 | NC | 625 | 100 | 3.1 | 0.5 TCNQ + 0.1 @ 30′ | 45′ | 97 | 1 | 2.5 | Nil |
| 3 | NC | 900 | 100 | 3.1 | 0.3 TCNQ + 0.2 @ 25′ | 45′ | 99 | 0 | 1.3 | Nil |
| 4 | NC | 1250 | 100 | 3.1 | 0.5 TCNQ + 0.15 @ 20′ | 25′ | 92 | 4 | 3.5 | Nil |
| 5 | NC | 1250 | 100 | 3.1 | 0.25 TCNQ | 30′ | 95 | 1 | 4.3 | Nil |
| 6 | NC | 1250 | 100 | 3.1 | 0.05 TCNQ | 30′ | 96 | 1 | 3.2 | 0.2 |
| 7 | NC | 1250 | 100 | 3.1 | 0.01 TCNQ | 30′ | 95 | 0 | 2.5 | 2.3 |
| 8 | NC | 1250 | 100 | 3.1 | 0.01 TCNQ + Air* | 30′ | 98 | 0 | 2.2 | Nil |
| 9 | NC | 1250 | 100 | 3.1 | 0.05 TCNQ + Air* | 31′ | 98 | 0 | 2.2 | Nil |
| 10 | NC | 1250 | 100 | 3.1 | 0.5 M Chloranil Air* | 35′ | 98 | 0 | 2.0 | 0.3 |
| 11 | NC | 1250 | 100 | 3.1 | 0.5 M TBHQ + $O_2$** | 30′ | 97 | 0 | 1.9 | 0.5 |
| 12 | NC | 1250 | 100 | 3.1 | 0.5 M TBHQ + Air* | 30′ | 95 | 2 | 2.5 | 0.7 |
| 13 | NC | 1250 | 100 | 3.1 | 0.5 M BHT + $O_2$** | 35′ | 95 | 0 | 3.1 | 1.8 |
| 14 | NC | 1250 | 100 | 3.1 | $O_2$** | 30′ | 93 | 1 | 2.0 | 3.3 |
| 15 | OC | 1250 | 100 | 1.0 | 0.05 TCNQ + Air* | 29′ | 72 | 20 | 7.4 | Nil |
| 16 | NC | 1250 | 150 | 0 | 0.5 M TCNQ | 25′ | 51 | 38 | 10 | Nil |
| 17 | NA | 625 | 150 | 3.1 | 0.5 M TCNQ<br>0.15 M TCNQ @ 30′ | 45′ | 94 | 0 | 2.4 | 3.5 |
| 18 | NC | 1250 | 100 | 3.1 | 0.1 M Chloranil + Air* | 40′ | 93 | 0 | 2.3 | 4.9 |
| 19 | SC | 900 | 100 | 3.1 | 0.5 M TCNQ + Air* | 50′ | 96 | 1 | 3 | Nil |
| 20 | SC | 900 | 100 | 3.1 | 0.05 M TCNQ + Air* | 50′ | 70 | 23 | 5.8 | Nil |
| 21 | SC | 900 | 100 | 3.1 | 0.5 M Chloranil + Air* | 50′ | 96 | 1 | 2.5 | Nil |
| 22 | SC | 1250 | 100 | 3.1 | None | 40′ | 65 | 23 | 2 | 10 |
| 23 | OC | 1250 | 100 | 3.1 | BHT + Air*** | 65′ | 95 | 0 | 1.8 | 3.3 |
| 24 | OC | 1250 | 100 | 3.1 | BHT | 30′ | 68 | 5 | 1.7 | 30 |

**Claims**

1. A process for preparing an alpha-chloro acyl chloride comprising contacting an acyl chloride with a chlorine source at a temperature of from 75°C to 125°C in the presence of

i) an effective amount of an auxiliary acidic agent and

ii) an effective amount of a free-radical inhibitor selected from food grade antioxidants, oxygen, a cyanoquinone material and mixtures thereof, wherein the acyl chloride is an acid chloride of the formula

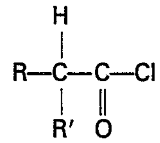

wherein R and R′ are H or the same or different hydrocarbyl substituents having from 1 to 28 carbon atoms; wherein the food grade antioxidant is selected from butylated hydroxyanisole, butylated hydroxytoluene, t-butyl hydroquinone, propyl gallate, 6-ethoxy-1,2-dihydro-2,2,4-trimethylquinoline, and mixtures thereof; and wherein the cyanoquinone material is selected from cyanoquinones, hexacyanobutadiene, and mixtures thereof.

2. A process according to Claim 1 wherein the chlorine source is elemental chlorine.

3. A process according to Claim 2 wherein the reaction temperature is 100°C to 110°C.

4. A process according to Claim 2 wherein the auxiliary acidic agent is selected from $PBr_3$, $PCl_3$, thionyl chloride, sulfuryl chloride, oxalyl chloride, sulfonyl chloride, $PCl_5$, phosgene, fluorosulfonic acid, chlorosulfonic acid, and trifluoromethanesulfonic acid, and mixtures thereof.

5. A process according to Claim 4 wherein the auxiliary acidic agent is chlorosulfonic acid.

6. A process according to Claim 4 or 5 wherein the reaction is conducted in the presence of oxygen.

7. A process according to Claim 1 wherein the cyanoquinone material is selected from 7,7,8,8-tetracyanoquinodimethane, hexacyanobutadiene, tetracyanonaphthoquinodimethane, and mixtures thereof.

8. A process according to Claim 7 wherein the cyanoquinone material is 7,7,8,8-tetracyanoquinodimethane.

9. A process according to Claims 7 or 8 wherein the reaction is conducted in the presence of oxygen.

## Patentansprüche

1. Verfahren zur Herstellung von alpha-Chlor-Acylchloriden, umfassend das Kontaktieren eines Acylchlorids mit einer Chlorquelle bei einer Temperatur von 75°C bis 125°C in Gegenwart von

i) einer wirksamen Menge eines aciden Hilfsmittels und

ii) einer wirksamen Menge eines Radikalfängers, ausgewählt aus Antioxidantien, die für Nahrungsmittel geeignet sind, Sauerstoff, einem Cyanochinonmaterial und deren Mischungen,

worin das Acylchlorid ein Säurechlorid der allgemeinen Formel

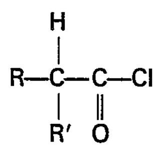

ist, worin R und R′ Wasserstoff bedeuten oder gleiche oder verschiedene Kohlenwasserstoffsubstituenten mit 1 bis 28 Kohlenstoffatomen sind, und worin das für Nahrungsmittel geeignete Antioxidans ausgewählt ist aus Butylhydroxyanisol, Hydroxy-toluol-butylether, t-Butylhydrochinon, Propylgallat, 6-Ethoxy-1,2-dihydro-2,2,4-trimethylchinolin und deren Mischungen, und worin das Cyanochinonmaterial ausgewählt ist aus Cyanochinonen, Hexacyanobutadien und deren Mischungen.

2. Verfahren gemäss Anspruch 1, worin die Chlorquelle elementares Chlor ist.

3. Verfahren gemäss Anspruch 2, worin die Reaktionstemperatur 100°C bis 100°C beträgt.

4. Verfahren gemäss Anspruch 2, worin das acide Hilfsmittel ausgewählt ist aus $PBr_3$, $PCl_3$, Thionylchlorid, Sulfurylchlorid, Oxalylchlorid, Sulfonylchlorid, $PCl_5$, Phosgen, Fluorsulfonsäure, Chlorsulfonsäure und Trifluormethansulfonsäure und deren Mischungen.

5. Verfahren gemäss Anspruch 4, worin das acide Hilfsmittel Chlorsulfonsäure ist.

6. Verfahren gemäss einem der Ansprüche 4 oder 5, worin die Reaktion in Gegenwart von Sauerstoff durchgeführt wird.

7. Verfahren gemäss Anspruch 1, worin das Cyanochinonmaterial ausgewählt ist aus 7,7,8,8-Tetracyanochinodimethan, Hexacyanobutadien, Tetracyanonaphthochinodimethan und deren Mischungen.

8. Verfahren gemäss Anspruch 7, worin das Cyanochinonmaterial 7,7,8,8-Tetracyanochinodimethan ist.

9. Verfahren gemäss Anspruch 7 oder 8, worin die Reaktion in Gegenwart von Sauerstoff durchgeführt wird.

## Revendications

1. Procédé de préparation d'un chlorure d'α-chloroacyle, comprenant la mise en contact d'un chlorure d'acyle avec une source de chlore, à une température de 75°C à 125°C, en présence

i) d'une quantité efficace d'un agent acide auxiliaire et

ii) d'une quantité efficace d'un inhibiteur de radicaux libres choisi parmi les antioxydants alimentaires,

l'oxygène, une substance cyanoquinone et leurs mélanges, dans lequel le chlorure d'acyle est un chlorure d'acide de formule

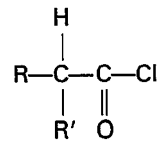

dans laquelle R et R' sont H ou des substituants hydrocarbonés identiques ou différents comportant de 1 à 28 atomes de carbone; l'antioxydant alimentaire étant choisi parmi l'hydroxyanisole butylé, l'hydroxytoluène butylé, la t-butylhydroquinone, le gallate de propyle, la 6-éthoxy-1,2-dihydro-2,2,4-triméthylquinoléine, et leurs mélanges; et la substance cyanoquinone étant choisi parmi les cyanoquinones, l'hexacyanobutadiène et leurs mélanges.

2. Procédé selon la revendication 1, dans lequel la source de chlore est le chlore élémentaire.

3. Procédé selon la revendication 2, dans lequel la température réactionnelle est de 100°C à 110°C.

4. Procédé selon la revendication 2, dans lequel l'agent acide auxiliaire est choisi parmi $PBr_3$, $PCl_3$, le chlorure de thionyle, le chlorure de sulfuryle, le chlorure d'oxalyle, le chlorure de sulfonyle, $PCl_5$, le phosgéne, l'acide fluorosulfonique, l'acide chlorosulfonique, l'acide trifluorométhanesulfonique, et leurs mélanges.

5. Procédé selon la revendication 4, dans lequel l'agent acide auxiliaire est l'acide chlorosulfonique.

6. Procédé selon la revendication 4 ou 5, dans lequel la réaction est réalisée en présence d'oxygène.

7. Procédé selon la revendication 1, dans lequel la substance cyanoquinone est choisie parmi le 7,7,8,8-tétracyanoquinodiméthane, l'hexacyanobutadiène, le tétracyanonaphtoquinodiméthane et leurs mélanges.

8. Procédé selon la revendication 7, dans lequel la substance cyanoquinone est le 7,7,8,8-tétracyanoquinodiméthane.

9. Procédé selon la revendication 7 ou 8, dans lequel la réaction est réalisée en présence d'oxygène.